(19) Europäisches Patentamt / European Patent Office / Office européen des brevets

(11) EP 2 432 766 B1

(12) # FASCICULE DE BREVET EUROPEEN

(45) Date de publication et mention de la délivrance du brevet: **17.07.2013 Bulletin 2013/29**

(21) Numéro de dépôt: **10728768.2**

(22) Date de dépôt: **17.05.2010**

(51) Int Cl.: ***C07D 235/26*** (2006.01) ***C07D 471/04*** (2006.01) ***A61K 31/4745*** (2006.01) ***A61P 35/00*** (2006.01)

(86) Numéro de dépôt international: **PCT/FR2010/050948**

(87) Numéro de publication internationale: **WO 2010/133794 (25.11.2010 Gazette 2010/47)**

(54) **COMPOSÉ ANTICANCÉREUX ET COMPOSITION PHARMACEUTIQUE LE CONTENANT**

ANTIKREBSVERBINDUNG UND PHARMAZEUTISCHE ZUSAMMENSETZUNG, DIE DIESE ENTHÄLT

ANTICANCER COMPOUND AND PHARMACEUTICAL COMPOSITION CONTAINING THE SAME

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**
Etats d'extension désignés:
**BA ME RS**

(30) Priorité: **18.05.2009 FR 0902392**

(43) Date de publication de la demande: **28.03.2012 Bulletin 2012/13**

(73) Titulaire: **SANOFI**
**75008 Paris (FR)**

(72) Inventeurs:
- **CARRY, Jean-Christophe** F-75013 Paris (FR)
- **CHEVE, Michel** F-75013 Paris (FR)
- **CLERC, François** F-75013 Paris (FR)
- **COMBEAU, Cécile** F-75013 Paris (FR)
- **GONTIER, Sylvie** F-75013 Paris (FR)
- **KRICK, Alain** F-75013 Paris (FR)
- **LACHAUD, Sylvette** F-75013 Paris (FR)
- **SCHIO, Laurent** F-75013 Paris (FR)

(74) Mandataire: **Nony et al**
**3, rue de Penthièvre**
**75008 Paris (FR)**

(56) Documents cités:
**WO-A-2007/012972**

- **M. S. COUMAR, C. H. A. CHEUNG, J.-Y. CHANG & H.-P. HSIEH: "Avances in Aurora kinase inhibitor patents" EXPERT OPINION ON THERAPEUTIC PATENTS, vol. 19, no. 3, mars 2009 (2009-03), pages 321-356, XP002543182**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention est relative au composé de formule (I) :

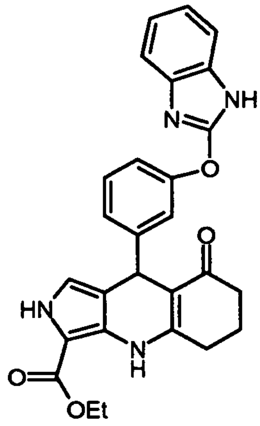

**(I)**

ainsi qu'à la composition pharmaceutique le contenant. De préférence, ce composé est le composé lévogyre **(Ia)**. Ce composé peut être utilisé en tant qu'anticancéreux. L'invention est aussi relative à un procédé d'obtention du composé **(I)** ou **(Ia)** ainsi qu'à certains des intermédiaires dudit procédé.

### [Problème technique]

Plusieurs stratégies pour traiter le cancer visent à inhiber les kinases du type Aurora, notamment Aurora A et B, qui sont impliquées dans la régulation de la mitose ; voir à ce propos Nature Reviews 2004, 4, 927-936 ; Cancer Res. 2002, 94, 1320 ; Oncogene 2002, 21, 6175 ; Mol.Cell.Biol. 2009, 29(4), 1059-1071 ; Expert Opin. Ther. Patents 2005, 15(9), 1169-1182 ; Clin.Cancer Res. 2008, 14(6), 1639).

Certains composés inhibiteurs d'Aurora (par exemple, **MLN-8237** de la société Millenium, **AZD-1152** de la société Astra-Zeneca ou **SNS-314** de la société Sunesis) sont actuellement évalués en essais cliniques. **MLN-8237** est sélectif vis d'Aurora A tandis que l'**AZD-1152** est sélectif vis à vis d'Aurora B. Les deux kinases, Aurora A et B, étant dérégulées dans le cancer, inhiber à la fois Aurora A et B procure un avantage par rapport à une inhibition sélective de l'une ou l'autre kinase. Par ailleurs, Il existe des composés multikinases tel que le composé de la société Astex, **AT-9283**, qui inhibent plusieurs kinases dont Aurora A et B. Il est difficile, pour ce type de composés, de prédire que l'inhibition des kinases Aurora pourra réellement être exploitée en clinique, car l'inhibition des autres kinases qu'Aurora A et B est susceptible d'engendrer des effets secondaires.

Un problème technique qu'entend résoudre l'invention est donc de mettre au point un composé qui soit un inhibiteur puissant et sélectif d'Aurora A et B.

L'enzyme phosphodiesterase à nucléotide cyclique PDE3 joue un rôle important dans la signalisation médiée par les nucléotides cycliques cAMP et cGMP qui a lieu dans les myocytes des muscles lisses cardiaques et vasculaires. L'inhibition de PDE3 par des petites molécules a une action inotrope et vasodilatatoire qui peut s'avérer utile à court terme pour le traitement de certaines cardiomyopathies présentant des défauts de contraction cardiaque. Toutefois, il a été montré que l'usage à long terme de ces molécules augmente la mortalité chez ce type de patients. En outre, l'utilisation d'inhibiteurs de PDE3 chez des patients ne présentant pas ce type de pathologie, tels que des patients atteints de cancer, peut entraîner des effets indésirables sur le rythme cardiaque. Il est donc important, dans le cadre d'une thérapie anticancéreuse, de ne pas inhiber PDE3. Voir à ce propos, Exp.Opin.Invest.drugs 2002, 11, 1529-1536 "Inhibitors of PDE3 as adjunct therapy for dilated cardiomyopathy" ; Eur. Heart J. supplements 2002, 4(supplement D), D43-D49 "What is wrong with positive inotropic drugs ? Lessons from basic science and clinical trials". Un autre problème technique qu'entend résoudre l'invention est que le composé inhibiteur d'Aurora A et B n'inhibe pas l'enzyme PDE3.

Il est important également que l'anticancéreux présente une stabilité métabolique (voir chap.10.2.2 de « Chimie pharmaceutique » G.L.Patrick, De Boeck, éd. 2003, isbn= 2-7445-0154-9). En effet, l'insuffisance de la pharmacocinétique de composés pharmaceutiques est une des raisons majeures de l'échec de leur développement (Curr. Pharm. 2005,11, 3545 "Why drugs fail-a study on side effects in new chemical entities"). De plus, le métabolisme est souvent un déterminant majeur de la clairance, des interactions médicamenteuses, de la variabilité intrindividuelle de la pharmacocinétique ainsi que de l'efficacité clinique et de la toxicité (Curr.Drug Metab. 2004, 5(5), 443-462 "Human hepatocytes in primary culture: the choice to investigate drug metabolism in man"). Un autre problème technique qu'entend

résoudre l'invention est que le composé inhibiteur d'Aurora A et B présente une bonne stabilité chimique et métabolique.

**[Art antérieur]**

Bioorg.Med.Chem.Lett. 2002, 2, 1481-1484 décrit dans le tableau II le composé 6A ayant une structure tricyclique différente.

WO 01/36422 décrit des composés ayant une structure tricyclique différente.

WO 2004/005323 décrit le composé E5A29 de formule (a) :

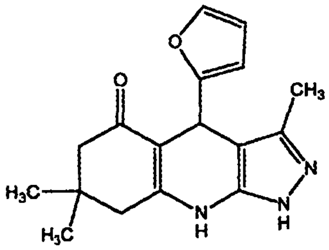

(a)

comme récepteur de l'EPO ayant une structure tricyclique différente. De plus, il ne comprend pas au sommet du tricycle de noyau phényle substitué par le groupe -O-benzimidazolyle.

WO 2005/016245 décrit des composés anticancéreux présentant une structure tricyclique différente, de formule (b) :

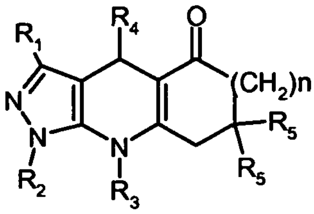

(b)

dans laquelle $R_4$ peut représenter un groupe phényle substitué. La substitution par le groupe -O-benzimidazolyle n'est pas décrite ni suggérée.

WO 2007/012972 et EP 1746097 décrivent des composés anticancéreux de formule (c)

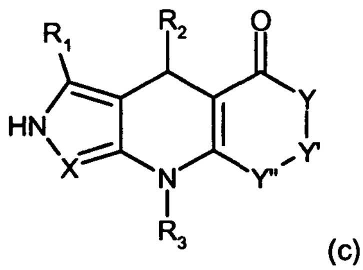

(c)

et selon un mode de réalisation, selon la formule (c') :

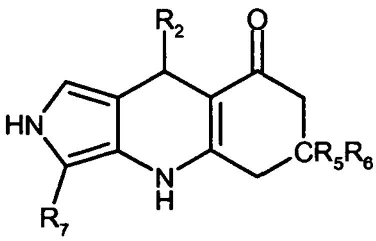

(c')

$R_2$ représente un groupe aryle ou hétéroaryle substitué, X représente N ou $CR_7$, $R_5$ et $R_6$ peuvent représenter tous les deux H ou $CH_3$. Aucun exemple de WO 2007/012972 ne comporte le groupe

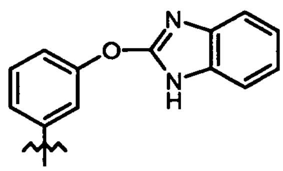

qui caractérise le composé de formule (I). De plus, parmi les composés qui ont été dédoublés, WO 2007/012972 enseigne que ce sont les composés dextrogyres qui sont les plus actifs sur Aurora A ou B (cf.ex.119 et 120 dans le tableau en page 147).

WO 02/062795 décrit des composés de formule (d) :

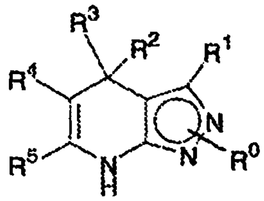

(d)

dans laquelle $R_4$ et $R_5$ peuvent éventuellement former un cycle à 5 ou 6 chaînons.

**[Brève description de l'invention]**

L'invention est relative au composé de formule (I) :

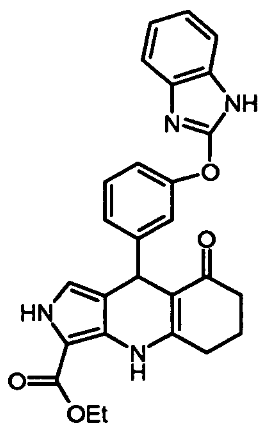

(I)

plus particulièrement sous sa forme lévogyre **(Ia)**, notamment celle présentant le pouvoir rotatoire $[\alpha]_D$= -38,6$\pm$0,7 à une concentration de 0,698 mg/ml dans le méthanol. Le composé peut exister sous forme de base ou d'un sel d'addition à un acide, notamment un acide pharmaceutiquement acceptable. Ce composé est un inhibiteur sélectif des kinases Aurora A et B. Il peut être utilisé en tant qu'anticancéreux.

L'invention est aussi realtive à une composition pharmaceutique comprenant le composé ainsi qu'au moins un excipient pharmaceutiquement acceptable et au médicament comprenant le composé.

L'invention est aussi relative au procédé d'obtention du composé consistant à :

■ faire réagir ensemble les 3 composés suivants, PG désignant un groupe protecteur de la fonction NH du benzimidazole

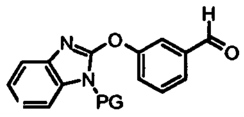 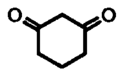 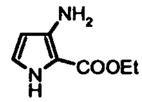

pour obtenir le composé :

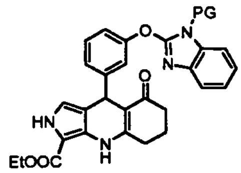 ;

■ à déprotéger la fonction NH du benzimidazole pour obtenir le composé de formule (I) ;
■ le cas échéant, à séparer le composé lévogyre.

La réaction entre les 3 composés est conduite dans un alcool au reflux, notamment le 1-butanol. Les intermédiaires suivants font aussi partie de l'invention :

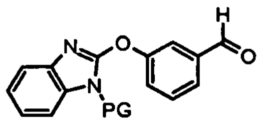 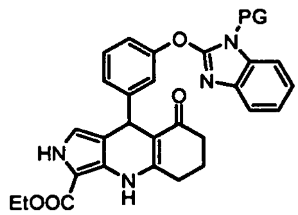 .

PG peut être par exemple le groupe

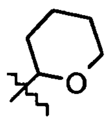 .

**[Description de l'invention]**

L'invention est relative au composé de formule (I) :

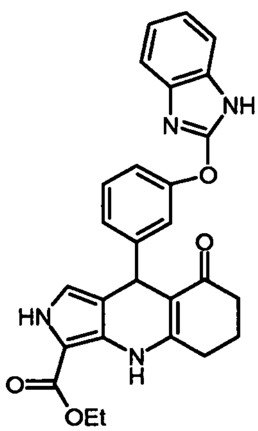

(I)

Ce composé peut exister sous forme racémique ou sous forme des deux énantiomères lévogyre **(Ia)** et dextrogyre **(Ib)**. Le composé lévogyre **(Ia)** présente une activité inhibitrice sélective sur les kinases Aurora A et B bien supérieure à celle de l'énantiomère dextrogyre **(Ib)**. Le composé lévogyre (la) présente également une activité antiproliférative supérieure à celle de l'énantiomère dextrogyre **(Ib)** (voir **Tableau I**).

Les trois composés **(I)**, **(Ia)** et **(Ib)** peuvent exister sous forme de base ou d'un sel d'addition à un acide. Le sel est avantageusement préparé avec un acide pharmaceutiquement acceptable (voir P.Stahl, C.Wermuth ; Handbook of Pharmaceutical Salts ; Wiley Ed. ISBN-13: 978-3906390260, ISBN-10: 3906390268), mais le sel d'un autre type d'acide, utile par exemple pour une étape de purification ou d'isolement fait également partie de l'invention.

Les composés **(I)** et **(Ia)** peuvent être utilisés comme anticancéreux ou pour la préparation d'un médicament pour le traitement d'un cancer. Le cancer est plus particulièrement un cancer dans lequel la/les kinases Aurora A et/ou B est/sont impliquée(s).
On obtient les composés **(I), (Ia)** et **(Ib)** selon le **Schéma 1** ci-dessous:

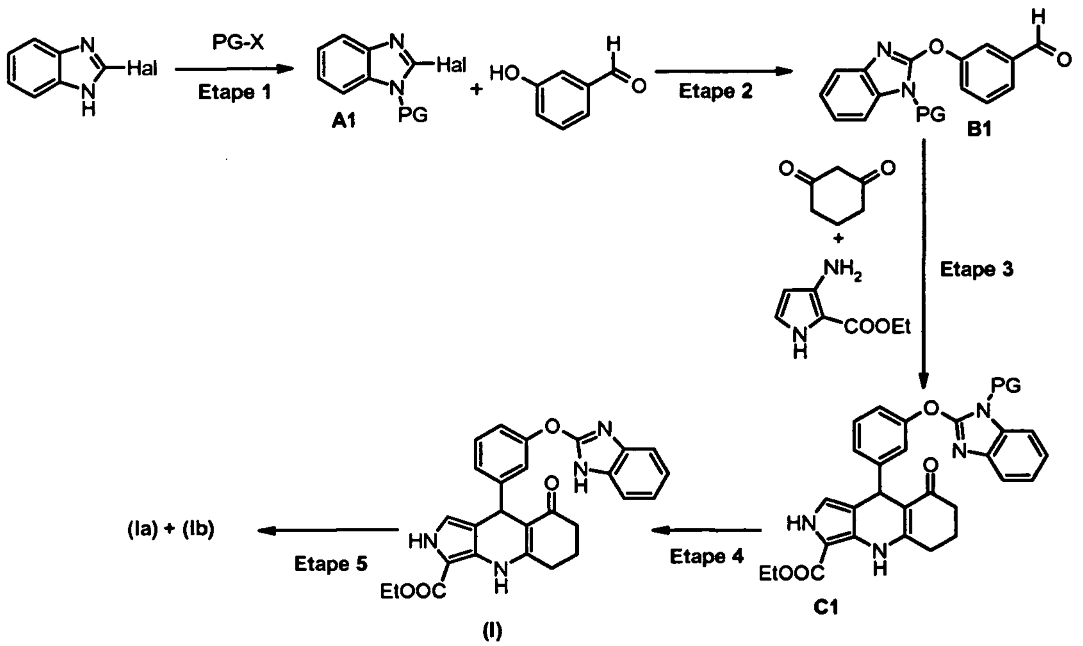


**Schéma I**

<u>étape 1 :</u> on protège la fonction NH d'un 2-halogéno-benzimidazole (Hal= Br ou Cl) à l'aide d'un groupe protecteur PG pour obtenir **A1**. PG-X représente un réactif permettant l'introduction du groupe protecteur PG. PG peut être plus particulièrement le dihydropyrane

( 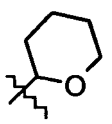 )

et dans ce cas, PG-X représente le 3,4-dihydro-2H-pyrane

( 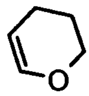 );

<u>étape 2 :</u> on fait réagir **A1** avec le 3-formyl-phénol en présence d'une base permettant d'obtenir l'ion phénolate correspondant pour obtenir **B1**. La base peut être un hydrure alcalin, comme par exemple NaH. La réaction est conduite dans un solvant polaire aprotique tel que le DMF ;
<u>étape 3 :</u> on fait réagir ensemble **B1**, le 3-amino-2-éthoxycarbonylpyrrole et la 1,3-cyclohexanedione, par ex. dans

un alcool (par ex. 1-butanol) au reflux, pour obtenir **C1** ; étape 4 : on déprotège la fonction NH de **C1** pour conduire au composé **(I)**. Les conditions de déprotection dépendent de la nature de PG. Par exemple, dans le cas où PG représente le dihydropyrane, on utilise un acide fort ;
étape 5: on sépare, par exemple à l'aide d'une chromatographie chirale, les deux énantiomères **(Ia)** et **(Ib)**.

Pour chacune des étapes 1-5, on pourra s'inspirer des conditions spécifiques décrites à l'exemple 1.

**[Exemples]**

**Méthodes analytiques**

**méthode LC/MS-A**

Les produits à analyser sont séparés sur une colonne UPLC modèle Aquity Beh C18 1,7 μm 2,1x50 mm (Waters) thermostatée à 70°C et éluée à un débit de 1 ml/min par un gradient d'acétonitrile contenant 0,1% d'acide formique (solvant B) dans l'eau contenant 0,1% d'acide formique (solvant A) programme d'élution : pallier isocratique à 5% de solvant B pendant 0,15 min, gradient de 5 à 100% de solvant B en 3,15 min puis retour aux conditions initiales en 0,1 min. Les produits sont détectés par un détecteur UV/vis à barrette de diode modèle PDA Acquity (Waters, gamme de longueurs d'ondes considérée 192-400 nm), un détecteur à diffusion de lumière modèle Sedex 85 (Sedere, gaz de nébulisation : azote, température de nébulisation : 32°C, pression de nébulisation 3,8 bar) et un spectromètre de masse Acquity SQD (Waters, fonctionnant en mode positif et négatif, gamme de masse considérée 80 à 800 uma).

**Méthode LC/MS-B**

Les spectres ont été obtenus sur un appareil Waters UPLC-SQD en mode d'ionisation électrospray positif et/ou négatif (ES+/-), dans les conditions de chromatographie liquide suivantes : colonne : ACQUITY BEH C18 1,7 μm, 2,1x50 mm ; $T_{colonne}$: 50°C ; débit : 1 ml/min ; solvants: A: $H_2O$ (0,1% acide formique) B: $CH_3CN$ (0,1% acide formique) ; gradient (2 mn) : 5 à 50% B en 0,8 min ; 1,2 min : 100% de B ; 1,85 min 100% de B ; 1,95 min 5% de B.

**$^1$H RMN**

Les spectres sont enregistrés sur un spectromètre de marque Brüker, le produit étant dissous dans le DMSO-d6. Les déplacements chimiques δ sont exprimés en ppm.

**IR**

Le spectre infrarouge est enregistré sur un spectromètre Nicolet Nexus en pastille de KBr avec une résolution de 2 $cm^{-1}$.

**Mesure du pouvoir rotatoire**

Les pouvoirs rotatoires ont été enregistrés sur un polarimètre 341 Perkin-Elmer.

**Analyse élémentaire**

Les analyses élémentaires ont été faites sur un analyseur EA1108 Thermo.

**Mesure de l'activité sur Aurora A et B**

La capacité à inhiber l'activité kinase de l'enzyme est estimée en mesurant l'activité kinase résiduelle de l'enzyme en présence de différentes concentrations du composé à tester (généralement de 0,17 à 10000 nM). Une courbe dose-réponse est établie et permet de déterminer une $IC_{50}$ (concentration inhibitrice à 50%). La mesure de l'activité kinase est réalisée par un dosage radioactif de la quantité de phosphate radioactif (33P) incorporé dans un fragment de la protéine NuMA (Nuclear Mitotic Apparatus protein) après 30 min d'incubation à 37°C. Le composé à tester est d'abord dissous à différentes concentrations dans du diméthylsulfoxyde (DMSO). La réaction s'effectue dans les puits d'une plaque de microtitration de type FlashPlate (Nickel Chelate FlashPlate-96, PerkinElmer). Chaque puits (100 μl) contient 10 nM d'Aurora A, 500 nM de NuMA, 1 μM ATP et 0,2 μCi d'ATP-γ-33P dans un tampon 50 mM Tris-HCl pH=7,5 ; 10 mM $MgCl_2$ ; 50 mM NaCl, 1 mM de dithiothréitol. Le pourcentage de DMSO final est de 3%. Après

homogénéisation par agitation, la plaque est incubée pendant 30 min à 37°C. Le contenu des puits est ensuite éliminé et les puits lavés par du tampon PBS. La radioactivité est ensuite mesurée à l'aide d'un compteur de type TRILUX 1450 Microbeta (WALLAC). Dans chaque plaque, 8 puits témoins sont réalisés : 4 témoins positifs (activité kinase maximale) pour lesquels la mesure est faite en présence d'enzyme et de substrat et en absence de composé de l'invention et 4 témoins négatifs (bruit de fond) pour lesquels la mesure est faite en absence d'enzyme, de substrat et de composé à tester. Les mesures sont données dans le **Tableau I**.

Aurora A

L'enzyme recombinante humaine Aurora A utilisée est exprimée sous forme entière avec un tag poly-Histidine en position N-terminale et produite dans E.Coli. Un fragment (acides aminés 1701-2115) de la protéine humaine NuMA avec un tag poly-Histidine en position C-terminale est exprimé sous forme recombinante dans E.coli.

Aurora B/Incenp

L'enzyme humaine Aurora B entière est coexprimée avec un fragment de la protéine humaine Incenp (aa 821-918) dans un système baculovirus et est exprimée en cellules d'insecte. Aurora B présente un tag poly-Histidine en position N-terminale tandis que le fragment Incenp possède un tag Glutathion-S-Transférase (GST) en position N-terminale. Les 2 protéines forment un complexe appelé Aurora B/Incenp. Un fragment (aa1701-2115) de la protéine humaine NuMA avec un tag poly-Histidine en position C-terminal est exprimé sous forme recombinante dans E.coli. Ce fragment est utilisé comme substrat.

**Mesure de la prolifération cellulaire**

Les cellules (lignée cellulaire tumorale HeLa réf: **ATCC CCL-2** et HCT116 réf: **ATCC CCL-247**) sont mises en présence du composé à tester durant 96 h et de la $^{14}C$-thymidine est ajoutée durant les dernières 24 h. La prolifération cellulaire est estimée par la quantité de $^{14}C$-thymidine incorporée dans les cellules.

Le composé à tester est mis en solution mère à 10 mM dans du DMSO, cette solution mère est utilisée pour réaliser une gamme de dilutions sérielles généralement de 10000 $\mu$M à 0,3 $\mu$M, ces dilutions sérielles sont elles-mêmes diluées au 1/50 dans le milieu de culture cellulaire (solution 20x) qui sera utilisée pour une dilution au 1/20 dans les plaques de culture cellulaire. Les concentrations finales du composé à tester seront généralement comprises entre 10000 et 0,3 nM.

<u>J0 :</u> les cellules ensemencées en plaques Cytostar 96 puits dans 180 $\mu$L de milieu de culture. Les plaques sont ensuite placées pendant 4 h dans un incubateur à 37°C 5% $CO_2$. Les produits à tester sont ensuite ajoutés dans un volume de 10 $\mu$L par puits en partant d'une solution 20x. Cette solution contient 2% de DMSO dans du milieu de culture. La concentration finale en DMSO est donc de 0,1%. Les plaques sont ensuite placées pendant 72 h dans un incubateur à 37°C / 5% $CO_2$.
<u>J3</u> : après 72 h, on ajoute 10 $\mu$L par puits de $^{14}C$-thymidine à 10 $\mu$Ci/mL dans du milieu de culture. Les plaques sont ensuite placées 24 h dans un incubateur à 37°C 5% $CO_2$.
<u>J4</u> : l'incorporation de $^{14}C$ thymidine est mesurée sur un compteur de radioactivité MicroBeta (Perkin-Elmer) après ces 24 h de « pulse ». La durée totale de traitement des cellules avec le produit à tester est de 96 h.

Le calcul des pourcentages d'inhibition IC50 se fait dans Excel grâce à la formule :

$$I\%\ Inhibition = 100 * (1 - (\frac{X - \ lanc}{TC - \ lanc}\ )$$

*X = Mesure pour l'échantillon*
*TC = Témoin Cellules*
*lanc = Mesure dans les puits sans Cellules*

Le calcul de $IC_{50}$ est fait avec le logiciel XLfit (IDBS, UK) à l'aide de la formule 205, avec le paramètre D (nombre de Hill) verrouillé à la valeur 1. Les résultats sont donnés dans le **Tableau I.**

**Evaluation de l'effet des composés de l'invention sur l'activité de l'enzyme PDE3**

L'effet des composés de l'invention sur l'activité de l'enzyme PDE3 a été évalué par la société CEREP (Le bois l'Evêque, 86600 Celle l'Evescault, France ; http://www.cerep.fr) selon son protocole standard (voir Bender, A.T., Beavo, J.A. Pharmacol Rev. 2006, 58, 488-520 : l'enzyme PDE3A est recombinante exprimée en cellules Sf9, le substrat est le cAMP et l'AMPc résiduel est mesuré par HTRF. L'inhibiteur de référence du test est la milrinone dont l'$IC_{50}$ est de 270 nM. Les % d'activité résiduels sont rapportés au contrôle sans inhibiteur). Les résultats sont exprimés soit en concentration induisant une inhibition de 50% ($IC_{50}$) soit en pourcentage d'inhibition mesuré à une concentration fixe du composé. Les résultats sont donnés dans le **Tableau I**.

**Mesure de la stabilité chimique des composés**

La stabilité chimique des composés a été mesurée dans différents milieux : acide chlorhydrique 0,05 N dans un mélange eau/acétonitrile 50/50 (v/v) ; soude 0,05 N dans un mélange eau/acétonitrile 50/50 (v/v) ; tampon phosphate de sodium 25 mM pH=7,4 dans un mélange eau/acétonitrile 50/50 (v/v) ; tampon phosphate de sodium 25 mM pH=7,4 dans un mélange eau/acétonitrile 50/50 (v/v) contenant 1% (p/v) de chlorhydrate de benzylamine ; tampon phosphate de sodium 25 mM pH=7,4 dans un mélange eau/acétonitrile 50/50 (v/v) contenant 1% (v/v) de 2-mercaptoéthanol. Les composés sont dilués dans les milieux d'étude à une concentration finale de 100 μM par dilution d'une solution mère à 10 mM dans le DMSO. Les solutions sont gardées à 20°C pendant un temps total de 48 h, et la concentration à des composés étudiés est mesurée au cours du temps (t=0, 1, 6, 12, 24 et 48 h) par HPLC. L'analyse HPLC est réalisée avec un appareil Agilent system 1100 équipé d'un détecteur à barrettes de diodes sur une colonne Luna C18, 30x4,6 mm, 3 μm (Phenomenex) éluée par un gradient d'acétonitrile (solvant B) dans l'eau contenant 0,5% (v/v) d'acide formique (solvant A) à un débit de 1,5 ml/min et une température de 25°C. Le programme d'élution est constitué d'un gradient de 10 à 90% de solvant B en 5 min suivi d'un pallier isocratique d'une minute à 90% de solvant B et retour aux conditions initiales en 1 min). La concentration des produits étudiés est estimée d'après la hauteur et l'aire du pic caractéristique du produit étudié sur un chromatogramme à la longueur d'onde maximale pour chaque produit. L'aire et la hauteur mesurées à chaque temps d'échantillonnage sont ramenées à l'aire et la hauteur obtenue pour l'échantillon au temps 0. Quand une dégradation est observée, un temps de demi-vie est mesuré à partir de la courbe temps-concentration obtenue. Les résultats sont donnés dans le **Tableau II**.

**Evaluation du métabolisme en présence de préparations microsomales de foies humains et murins.**

Si les préparations de microsomes restent importantes pour la détermination de la stabilité métabolique d'un composé pharmaceutique, la culture primaire d'hépatocytes permet une évaluation plus fine de sa clearance intrinsèque et de meilleures corrélations vitro-vivo permettant d'anticiper la clearance hépatique chez l'homme.

Les composés de l'invention (5 μM) sont incubés à température physiologique sur des fractions microsomales de foie humaines et murines (1 mg/ml de protéines), dilué dans un tampon phosphate, en présence de sérum albumine bovine (1 mg/ml BSA), et de nicotinamide adénine dinucléotide phosphate, forme réduite (1 mM NADPH). Pour terminer l'incubation, 4 volumes d'acétonitrile contenant de la corticostérone comme standard interne (IS) sont ajoutés. Les échantillons sont centrifugés et les surnageant sont analysés par couplage chromatographie liquide/spectrométrie de masse en tandem (LC/MS-MS). L'analyse LC/MS-MS est réalisée sur un spectromètre de masse AP14000-QTRAP (Sciex) équipé d'un système chromatographique 1100 series (Agilent) et d'un injecteur automatique Pal CTC. Les données sont aquises et analysées à l'aide d'un logiciel Analyst 1.4.1. Les échantillons sont séparés sur une colonne Polaris C18, 3 μm éluée à un débit de 0,7 ml/min par un gradient d'acétonitrile (solvant B) dans l'eau contenant 0,1% d'acide formique (solvant A). Le programme d'élution est constitué du gradient de 20 à 90% de solvant B en 2 min, d'un pallier isocratique à 90% de solvant B de 0,9 min et d'un retour aux conditions initiales en 0,1 min. L'aire des pics chromatographique du composé et de l'étalon interne sont intégrés à l'aide de l'algorithme Analyst-Classic. La stabilité métabolique des produits de l'invention est estimée par comparaison des ratios d'intégrations (courants ioniques des composés/ courant ionique IS) mesurés après 0 min (t0) et 20 min (t20) d'incubation. La stabilité métabolique est alors exprimée en pourcentage de disparition selon la formule suivante :

$$\text{Métabolisme\%} = (\text{ratio des pics à t0} - \text{ratio des pics à t20}) / \text{ratio des pics à t0}$$

Les résultats sont donnés dans le **Tableau III**.

**Evaluation de la clairance en présence d'hépatocytes humains.**

Les composés de l'invention (0,5 ou 5 μM) sont incubés pendant 24 h dans des plaques 48 puits recouverts de collagène en présence d'hépatocytes humains frais ou cryoconservés (-200000 cellules/puits) issus de donneurs spécifiques dans un incubateur à une température physiologique. Les incubations sont conduites avec un milieu de culture (HAM F12 - William E). A différent temps (0 ; 0,5 ; 1 ; 2 ; 4 ; 6 ; 8 et 24 h), 100 μl de chaque puits sont prélevés et la cinétique est stoppée par addition de 700 μl d'un mélange d'acétonitrite/eau 70/30 (v/v) contenant de la corticostérone comme standard interne (IS). Les cellules sont ensuite dissociées et les milieux intra- et extracellulaire mélangés et stockés congelés à -20°C jusqu'à leur analyse. Après décongélation les échantillons sont centrifugés à 300 g pendant 20 min et les surnageants sont analysés par couplage chromatographie liquide/spectrométrie de masse en tandem (LC/MS-MS). L'analyse LC/MS-MS est réalisée sur un spectromètre de masse AP14000-QTRAP (Sciex) équipé d'un système chromatographique 1100 series (Agilent) et d'un injecteur automatique Pal CTC. Les données sont aquises et analysées à l'aide d'un logiciel Analyst 1.4.1. Les échantillons sont séparés sur une colonne Polaris C18, 3 μm éluée à un débit de 0,7 ml/min par un gradient d'acétonitrile (solvant B) dans l'eau contenant 0,1% d'acide formique (solvant A). Le programme d'élution est constitué du gradient de 20 à 90% de solvant B en 2 min, d'un pallier isocratique à 90% de solvant B de 0,9 min et d'un retour aux conditions initiales en 0,1 min. La concentration des produits de l'invention est mesurée par intégration du courant ionique des ions caractéristiques des produits rapportés à l'étalon interne (IS). Les ratios composé/IS obtenus sont rapportés à des standards de calibrations de concentrations connues, permettant ainsi la détermination de concntration des produits de l'invention. La clairance intrinsèque (exprimée en $ml.h^{-1}.10^{-6}$ cellules) est alors déterminée à partir des profils cinétiques (concentrations/temps) en utilisant le logiciel WinNonLin (5.0). Les résultats sont donnés dans le **Tableau IV**.

**Exemple 1 : préparation du 8-oxo-9-[3-[1H-benzimidazol-2-yloxyl-phényl]-4,5,6,7,8,9-hexahydro-2H-pyrrolo [3,4-b]quinoline-3-carboxylate d'éthyle (I)**

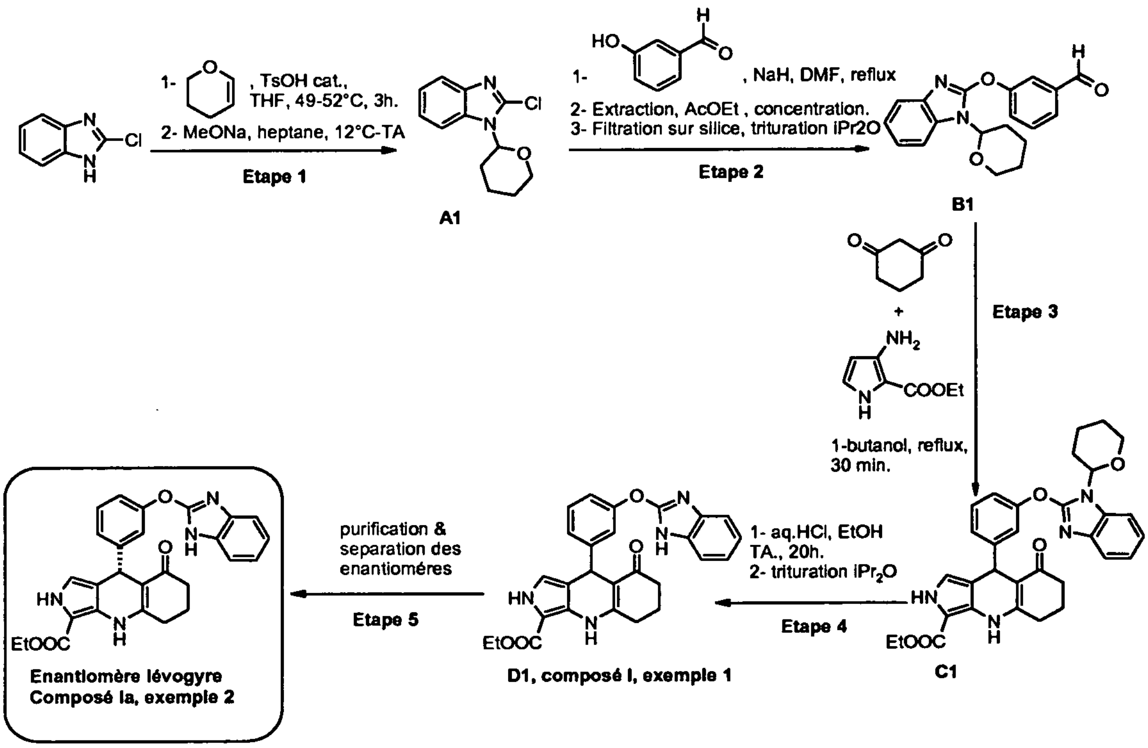


**A1. 2-Chloro-1-(tétrahydro-pyran-2-yl)-1 H-benzimidazole (CAS N° 208398-29-2)**

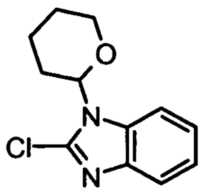

Dans un réacteur de 10 l, sous argon et sous agitation, on charge 2,5 l de THF, 180 g de 2-chlorobenzimidazole (1,18 moles) et 325 ml de 3,4-dihydro-2H-pyrane (6,56 moles, 3 éq.). Le réacteur est chauffé jusqu'à solubilisation (température du milieu : 40°C). On introduit ensuite 6,3 g d'acide para-toluènesulfonique (0,033 mole, 0,028 éq.). La température est maintenue entre 49 et 52 °C pendant 2,5 h. On refroidit à 12°C et on ajoute 7,65 g de méthylate de sodium (0,142 moles, 0,12 éq.) en maintenant l'agitation sur une durée totale de 15 min. La température est ensuite portée à 18°C, 5 l de n-heptane sont ajoutés et l'ensemble est filtré sur 300 g de Clarcel FLO-M, le rétentat est lavé par 5 l -de n-heptane. Le-filtrat est-concentré à sec sous pression réduite et on obtient 292,6 g de 2-chloro-1-(tetrahydro-pyran-2-yl)-1H-benzimidazole sous forme d'une huile légèrement jaune (rendement quantitatif). RMN $^1$H (400 MHz, DMSO-d6) : 1,42 à 2,01 (m, 5H); 2,21 à 2,34 (m, 1 H); 3,69 à 3,78 (m, 1 H); 4,12 (d, *J*=11,4 Hz, 1 H); 5,72 (dd, *J*=2,4 et 11,2 Hz, 1 H); 7,22 à 7,34 (m, 2H); 7,62 (d, *J*=7,2 Hz, 1 H); 7,78 (d, *J*=7,2 Hz, 1 H).

**B1 : 3-[1-(Tétrahydro-pyran-2-yl)-1H-benzimidazol-2-yloxy]-benzaldéhyde**

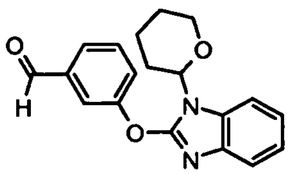

Dans 2 ballons tricol de 2 l, équipés chacun d'un réfrigérant, d'un thermomètre et d'un arbre d'agitation, sous argon, on charge du N,N-diméthylformamide (0,4 l par ballon), et du 3-hydroxybenzaldéhyde (68,5 g, ballon 1 ; 64,2 g, ballon 2 ; 1,08 moles). De l'hydrure de sodium (dispersion à 60% dans l'huile minérale) est ensuite ajouté par portions (ballon 1: 26 g ; ballon 2: 24 g ; 1,25 moles, 1,2 éq.), la température maximale lors de l'addition est de 32°C. Le 2-chloro-1-(tétrahydro-pyran-2-yl)-1H-benzimidazole (A1), titre estimé à 85%) est ensuite introduit (ballon 1: 151 g dans 0,5 l de N,N-diméthylformamide ; ballon 2: 142 g dans 0,5 l de N,N-diméthylformamide; 1,05 moles, 0,97 éq.). Le milieu est ensuite porté au reflux (température 140°C, temps de montée en température 40 min) et le reflux est maintenu pendant 1 h. Le chauffage est ensuite arrêté et le milieu est laissé à refroidir pendant 1,5 h. Le contenu des 2 ballons est rassemblé. Le tout est mélangé lentement à 5 l d'eau glacée. La phase aqueuse obtenue est ensuite extraite par 4x2,5 l d'acétate d'éthyle (AcOEt). Les phases organiques sont ensuite rassemblées, lavées par 3 l d'eau puis 2 l d'une solution saturée NaCl et enfin séchées par addition $MgSO_4$ pendant une nuit. La phase organique obtenue est ensuite filtrée sur un verre fritté (porosité 4) et concentrée à sec sous pression réduite pour obtenir 385 g d'une huile marron (LC/MS-A, tr (temps de rétention)=1,86 min, MS mode positif : m/z=323,16).

Une fraction de 158 g du matériel brut obtenu précédemment est solubilisée à chaud dans 1,5 l d'un mélange n-heptane/AcOEt (8/2 en volumes), combiné à 500 g de silice (70-30 mesh) et l'ensemble est agité pendant 45 min. La suspension obtenue est filtrée sur Célite, lavée par 3 l d'un mélange n-heptane/AcOEt (8/2 en volumes). La phase organique obtenue est concentrée à sec sous pression réduite. Le résidu est remis en suspension dans 200 ml d'éther isopropylique par agitation mécanique et traitement aux ultrasons puis filtré sur verre fritté (porosité 3). Le solide obtenu est lavé par 2x40 ml d'éther ispropylique et séché sous pression réduite à 40°C pendant 16 h pour donner 68 g de solide. Un traitement similaire appliqué au reste du brut permet d'obtenir 87,8 g de solide. Les solides obtenus sont réunis et homogénéisés pour obtenir 155,8 g de 3-[1-(tétrahydro-pyran-2-yl)-1 H-benzimidazol-2-yloxy]-benzaldéhyde sous forme de cristaux beige clair (LC/MS-A, tr=1,87 min, MS mode positif m/z=323,13). MS(LC/MS-B): tr=1,00 min; [M+H]+ : m/z 323 ; RMN $^1$H (400 MHz, DMSO-d6) : 1,54 à 1,62 (m, 1H); 1,63 à 1,84 (m, 2H); 1,92 à 2,03 (m, 2H); 2,30 à 2,42 (m, 1 H); 3,70 à 3,79 (m, 1 H); 4,10 (d, *J*=11,5 Hz, 1 H); 5,74 (dd, *J*=2,1 et 11,1 Hz, 1H); 7,13 à 7,22 (m, 2H); 7,43 (d, *J*=7,3 Hz, 1H); 7,65 (d, *J*=7,3 Hz, 1H); 7,73 (t, *J*=7,8 Hz, 1 H); 7,78 à 7,83 (m, 1 H); 7,87 (d, *J*=7,8 Hz, 1 H); 7,96 (s, 1 H); 10,05 (s, 1 H).

Le lavage des phases silices utilisées précédemment par 2 l d'un mélange n-heptane/AcOEt (1/1 en volumes) permet d'obtenir 67 g de matériel après concentration à sec sous pression réduite. Ce matériel est repris par 2 l d'un

mélange n-heptane/AcOEt (9/1 en volumes), combiné avec 285 g de silice (70-30 mesh), agité et traité par ultrasons pendant 1 h. La suspension est ensuite filtrée sur Célite, la phase solide est lavée par 2 l d'un mélange n-heptane/AcOEt (9/1 en volumes). Le filtrat est concentré à sec sous pression réduite et le résidu est trituré dans 400 ml d'un mélange n-heptane/éthanol (95/5 en volumes), filtré sur verre fritté (porosité 3) et séché sous pression réduite pour donner 35 g de 3-[1-(tetrahydro-pyran-2-yl)-1H-benzimidazol-2-yloxy]-benzaldéhyde sous forme de cristaux beige clair. (LC/MS-A, tr=1,93 min, MS mode positif m/z=323,16).

**C1: 8-Oxo-9-{3-[1-(tetrahydro-pyran-2-yl)-1H-benzimidazol-2-yloxy]-phényl}-4,5,6,7,8,9-hexahydro-2H-pyrrolof3,4-blauinoline-3-carboxylate d'éthyle**

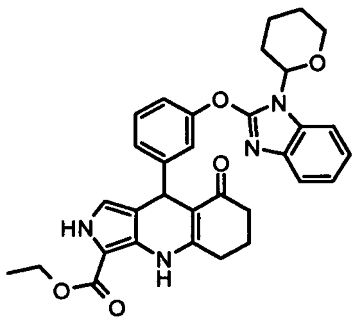

Dans un Erlenmeyer de 2 l sous agitation magnétique, on ajoute 50 g de chlorhydrate de 3-amino-2-éthoxycarbonylpyrrole et 0,204 l de soude 2N. L'ensemble est agité pendant 15 min à température ambiante (TA), puis il est ensuite extrait par 3x0,3 l de dichlorométhane. Les phases organiques sont rassemblées, séchées sur $MgS0_4$ et concentrée à sec sous pression réduite. Le résidu est trituré avec du n-pentane, filtré et séché sous pression réduite jusqu'à poids constant pour donner 36,4 g de 3-amino-2-éthoxycarbonylpyrrole sous forme d'un solide marron.

Dans un ballon tricol de 2 l muni d'un arbre d'agitation, d'un thermomètre et d'un réfrigérant, on charge 1,2 l de 1-butanol, 145 g de 3-[1-(tetrahydro-pyran-2-yl)-1H-benzimidazol-2-yloxy]-benzaldéhyde (0,405 mole, B1), 62,4 g de 3-amino-2-éthoxycarbonylpyrrole (1 éq., 0,405 mole), 46,8 g de 1,3-cyclohexanedione à 97% (1 éq., 0,405 mole) et 70,5 ml de N,N-diisopropyléthylamine (1 éq.) et on porte au reflux (temps de montée en température 55 min, reflux maintenu 30 min, température 114°C). Le milieu est ensuite refroidi à TA et concentré à sec sous pression réduite pour donner 290 g d'une huile marron contenant le 8-oxo-9-{3-[1-(tetrahydro-pyran-2-yl)-1H-benzimidazol-2-yloxy]-phényl}-4,5,6,7,8,9-hexahydro-2H-pyrrolo[3,4-b]quinoline-3-carboxylate d'éthyle. (LC/MS-A, tr=1,96 min, MS mode positif m/z=553,33). Une opération similaire conduite avec 35 g de 3-[1-(tetrahydro-pyran-2-yl)-1H-benzimidazol-2-yloxy]-benzaldéhyde (0,098 mole, exemple B1) permet d'obtenir 72 g d'une huile marron contenant le 8-oxo-9-{3-[1-(tetrahydro-pyran-2-yl)-1H-benzimidazol-2-yloxy]-phényl}-4,5,6,7,8,9-hexahydro-2H-pyrrolo[3,4-b]quinoline-3-carboxylate d'éthyle. (LC/MS-A, tr=1,96 min, MS mode positif m/z=553,35).

**D1 : 8-Oxo-9-[3-(1H-benzimidazol-2-yloxy)-phényl]-4,5,6,7,8,9-hexahydro-2H-pyrrolo[3,4-b]quinoline-3-carboxylate d'éthyle (composé I)**

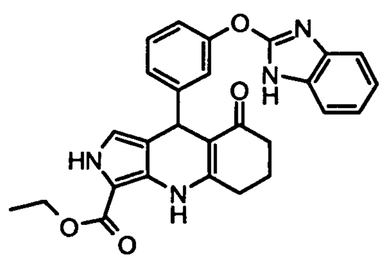

Dans un ballon de 2 l, on charge 224 g de l'huile marron contenant le 8-oxo-9-{3-[1-(tetrahydro-pyran-2-yl)-1H-benzimidazol-2-yloxy]-phényl}-4,5,6,7,8,9-hexahydro-2H-pyrrolo[3,4-b]quinoline-3-carboxylate d'éthyle (ex.1.3), 0,7

l d'éthanol et 0,243 l d'acide chlorhydrique 2N. Le mélange est agité 16 h à TA, puis filtré sur verre fritté (porosité 4). Le filtrat est concentré à sec sous pression réduite et le résidu est trituré avec 0,5 l d'éther isopropylique. Le solide obtenu est séché sous pression réduite jusqu'à poids constant pour donner 253 g d'un solide marron contenant le 8-oxo-9-[3-(1H-benzimidazol-2-yloxy)-phényl]-4,5,6,7,8,9-hexahydro-2H-pyrrolo[3,4-b]quinoline-3-carboxylate d'éthyle. (LC/MS-A, tr=1,46 min, MS mode positif m/z=469,29). Une opération similaire conduite avec 54 g de l'huile marron contenant le 8-oxo-9-{3-[1-(tetrahydro-pyran-2-yl)-1H-benzimidazol-2-yloxy]-phényl}-4,5,6,7,8,9-hexahydro-2H-pyrrolo [3,4-b]quinoline-3-carboxylate d'éthyle (C1) permet d'obtenir 60 g d'un solide marron contenant le 8-oxo-9-[3-(1 H-benzimidazol-2-yloxy)-phényl]-4,5,6,7,8,9-hexahydro-2H-pyrrolo[3,4-b]quinoline-3-carboxylate d'éthyle. (LC/MS-A, tr=1,48 min, MS mode positif m/z=469,29).

Une fraction aliquote de 0,8 g du matériel obtenu peut être purifiée par chromatographie sur une cartouche de 50 g de silice (10-90 μm) (Biotage SNAP, KP-Sil) éluée par un palier isocratique de dichlorométhane de 20 min, puis un gradient de 0 à 1% en volumes d'isopropanol dans le dichlorométhane en 1 h et enfin un pallier isocratique de dichlorométhane/isopropanol (99/1 en volumes) de 20 min. Les fractions contenant le produit attendu sont réunies pour donner 0,21 g d'un solide jaune. Les produits de 2 séparations chromatographiques similaires conduites à la même échelle sont cristallisés dans l'acétonitrile pour donner au total 0,16 g de 8-oxo-9-[3-(1H-benzimidazol-2-yloxy)-phényl]-4,5,6,7,8,9-hexahydro-2H-pyrrolo[3,4-b]quinoline-3-carboxylate d'éthyle sous forme de cristaux beiges. (LC/MS-A, tr=1,61 min, MS mode positif m/z=469,28). RMN $^1$H (400 MHz, DMSO-d6) : 1,29 (t, *J*=7,0 Hz, 3H); 1,80 à 1,97 (m, 2H); 2,19 à 2,27 (m, 2H); 2,55 à 2,69 (m, 1 H); 2,81 (dt, *J*=4,8 et 17,2 Hz, 1 H); 4,26 (q, *J*=7,0 Hz, 2H); 5,11 (s, 1 H); 6,73 (d, *J*=3,3 Hz, 1 H); 7,02 à 7,16 (m, 5H); 7,25 (t, *J*=7,9 Hz, 1 H); 7,31 à 7,38 (m, 2H); 8,34 (s, 1 H); 11,33 (s large, 1 H); 12,26 (s large, 1 H). Analyse élémentaire : C= 68,72% ; H= 5,10% ; N= 11,82% ; $H_2O$= 0,38%.

**Exemple 2: Enantiomère lévogyre (Ia) du 8-oxo-9-[3-(1H-benzimidazol-2-yloxy)-phényl]-4,5,6,7,8,9-hexahydro-2H-pyrrolo[3,4-b]quinoline-3-carboxylate d'éthyle**

L'énantiomère lévogyre est purifié à partir du produit brut de l'exemple D1 sur une colonne chirale Welk-01RR, 10 μM, 80x350 mm (Regis, USA) éluée par un mélange n-heptane/dichlorométhane/éthanol/triéthylamine (50/47,5/2,5/0,1 en volumes). L'élution des produits est détectée par spectroscopie UV à 265 nm. Des quantités de 10 g du produit brut décrit dans l'exemple D1 sont injectées lors de chaque opération. Dans ces conditions, le pic correspondant à l'énantiomère lévogyre est élué avec un tr entre 50 et 80 min. Les fractions d'énantiomère lévogyre purifié correspondant aux opérations nécessaires pour purifier 310 g du produit brut décrit dans l'exemple D1, sont rassemblées, homogénéisées et concentrées à sec sous pression réduite pour fournir 50 g d'un solide beige. Spectre de masse (LC/MS-B): tr=0,77 min ; [M+H]+ : m/z 469 ; [M-H]- : m/z 467. RMN $^1$H (400 MHz, DMSO-d6) : 1,29 (t, *J*=7,1 Hz, 3H); 1,79 à 1,97 (m, 2H); 2,19 à 2,27 (m, 2H); 2,55 à 2,66 (m, 1 H); 2,81 (dt, *J*=4,9 et 17,1 Hz, 1 H); 4,26 (q, *J*=7,1 Hz, 2H); 5,12 (s, 1 H); 6,73 (d, *J*=3,4 Hz, 1H); 7,02 à 7,16 (m, 5H); 7,25 (t, *J*=8.3 Hz, 1H); 7,29 à 7,41 (m, 2H); 8,32 (s, 1H); 11,31 (s large, 1 H); 12,26 (s large, 1H). IR : principales bandes : 1678 ; 1578 ; 1525 ; 1442 ; 1188 ; 1043 et 743 $cm^{-1}$. Pouvoir rotatoire : $[\alpha]_D$= -38,6±0,7 à C=0,698 mg/ml dans le méthanol. Analyse élémentaire : C= 68,18% ; H= 5,92% ; N= 11,22%; $H_2O$= 1,25%.

**Exemple 3 : Enantiomère dextrogyre (Ib) du 8-oxo-9-[3-(1H-benzimidazol-2-yloxy)-phényl]-4,5,6,7,8,9-hexahydro-2H-pyrrolo[3,4-b]quinoline-3-carboxylate d'éthyle**

L'énantiomère dextrogyre est obtenu par purification du produit purifié de l'exemple D1 par chromatographie sur une colonne chirale Welk-01 SS, 10 μM, 60x350 mm (Regis, USA) éluée par un mélange n-heptane/éthanol (7/3 puis 6/4 en volumes). L'élution des produits est détectée par spectroscopie UV. Les fractions de l'énantiomère dextrogyre sont rassemblées, homogénéisées et concentrées à sec sous pression réduite pour fournir 1,9 g d'une poudre jaune. MS (LC/MS-B) : tr=0,77 min ; [M+H]+ : m/z=469,2 ; [M-H]- : m/z=467,2. Pouvoir rotatoire : $[\alpha]_D$= +53,1±1,1 à C=3,6 mg/ml dans le méthanol.

**Exemples 4-13 :** les composés des exemples 4-11 ont été préparés selon l'enseignement de WO 2007/012972 (voir procédé de la rev.26). Les produits dihydropyridines tricycliques de formule (II) peuvent être préparés selon le **Schéma II** :

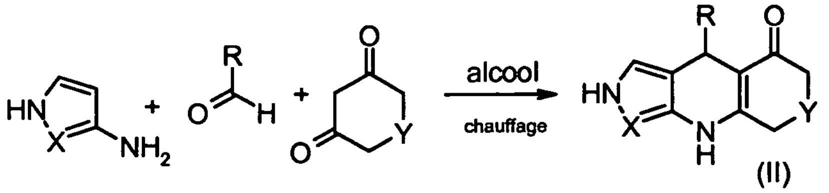

(II)

**Schéma II**

Un mélange de 1 équivalent de pyrazole (X=N) ou de pyrrole-2-carboxylate (X=COOEt), 1 équivalent d'aldéhyde R-CHO et 1 équivalent de dérivé dicétone (Y=$CH_2$, $CMe_2$, N-Boc) est chauffé à reflux dans un alcool tel que l'éthanol ou le 1-butanol pendant une période comprise entre ½ h et plusieurs h. Le mélange est ensuite refroidi à température ambiante. Les produits désirés sont isolés par filtration ou bien le solvant est évaporé à sec. Si nécessaire, le produit brut est purifié par chromatographie sur gel de silice ou bien par chromatographie liquide préparative haute performance (HPLC).

Lorsque Y représente N-Boc, les produits peuvent être déprotégés en utilisant une solution d'acide trifluoroacétique dans le dichlorométhane (50/50) ou bien une solution d'acide chlorhydrique dans le dioxanne (**Schéma III**) :

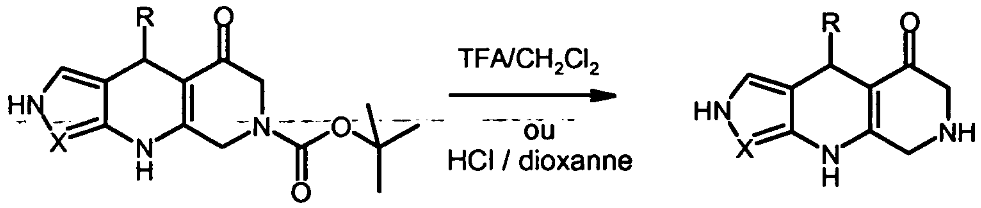


**Schéma III**

Les aldéhydes de formule générale (III), utilisés dans la préparation du composé 4 (R=H), peuvent être obtenus selon le **Schéma IV**. R désigne un (n=1) ou plusieurs substituants (n allant de 2 à 4) du noyau benzimidazole choisis parmi : H, F, Cl, Br, OH, SH, $CF_3$, $OCF_3$, $OCH_3$, $SCF_3$, $SCH_3$, $OCHF_2$, $OCH_2F$, $SCH_2F$, ($C_1$-$C_6$)alkyle, O-allyle, phényle éventuellement substitué par un ou plusieurs atome(s) d'halogène.

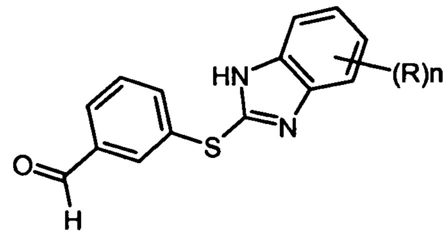

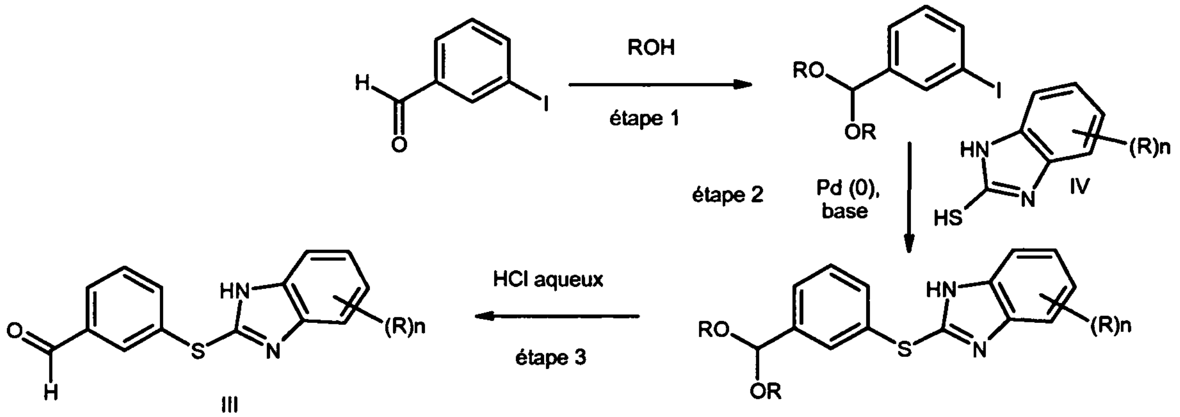


**Schéma IV**

<u>étape 1 :</u> on protège la fonction aldéhyde du 3-iodo-benzaldéhyde à l'aide d'un groupement protecteur alcool et plus particulièrement diol (l'éthylène glycol par exemple) en présence d'un acide tel que l'acide para-toluènesulfonique dans un solvant inerte tel que le toluène et à une température comprise entre 20°C et la température d'ébullition du milieu réactionnel;

étape 2 : on fait réagir l'intermédiaire formé avec un produit de formule (IV) en présence d'un complexe de palladium tel que le bis(dibenzylidèneacétone)palladium, d'un dérivé de phosphine tel que le bis[(2-diphénylphosphino)phényl] éther et d'une base telle que le tert-butylate de sodium, dans un solvant inerte tel que le toluène et à une température comprise entre 20°C et la température d'ébullition du milieu réactionnel ;

étape 3 : on déprotège la fonction aldéhyde en présence d'une solution aqueuse d'acide tel que l'acide chlorhydrique, éventuellement dans un solvant tel que l'acétone et à une température comprise entre 20°C et la température d'ébullition du milieu réactionnel.

Dans le **Tableau I**, sont comparées les activités d'inhibition sur Aurora A et B, les activités antiprolifératives sur les lignées HeLa et HCT116, l'activité d'inhibition de PDE3 ainsi que le métabolisme. On constate que le composé **(I)** ou **(Ia)** présente une forte inhibition des kinases Aurora A et B ainsi qu'une très bonne activité sur les lignées HeLa et HCT116.

**Tableau I**

| exemple | composé | IC50 AuroraB/ Incenp [nM] | IC50 Aurora A [nM] | IC50 HeLa [nM] | IC50 HCT116 [nM] | IC50 PDE3 [nM] | microsome humain [%] | microsome souris [%] |
|---|---|---|---|---|---|---|---|---|
| **1 selon l'invention** | 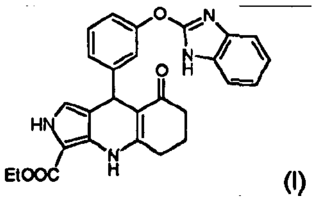 (I) | 4 | 6 | 205 | nd | 78% Inhibition à 1000 nM | 37% | 31% |
| **2 - selon** | composé lévogyre (Ia) | 1 | 1 | 67 | 20 | 4000 | 32% | 50% |
| **3 - comparatif** | composé dextrogyre (Ib) | 900 | 3800 | >10000 | nd | nd | nd | nd |
| **4 - comparatif** | 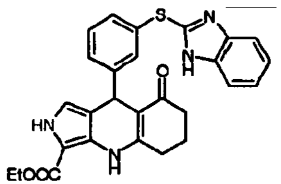 | 6 | 72 | 9664 | nd | nd | 77% | 67% |
| **5 - comparatif** | 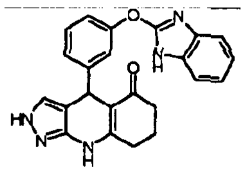 | 6 | 23 | 9633 | nd | nd | nd | nd |
| **6 - comparatif** | 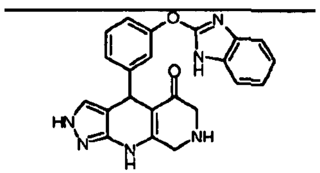 | 13 | 109 | >10000 | nd | nd | nd | nd |

(suite)

| exemple | composé | IC50 AuroraB/ Incenp [nM] | IC50 Aurora A [nM] | IC50 HeLa [nM] | IC50 HCT116 [nM] | IC50 PDE3 [nM] | microsome humain [%] | microsome souris [%] |
|---|---|---|---|---|---|---|---|---|
| 7 - comparatif | 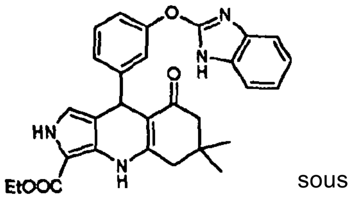 sous forme de trifluoroacétate | 4 | 3 | 430 | nd | nd | 65% | 96% |
| 8 - comparatif | 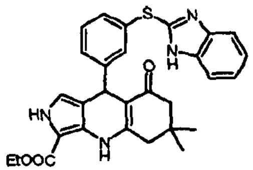 | 21 | 102 | 6863 | nd | nd | 80% | 84% |
| 9 - comparatif | 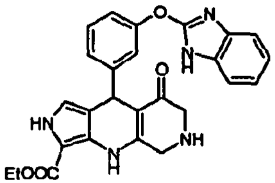 | 8 | 15 | 9 462 | nd | nd | nd | nd |
| 10 - comparatif | 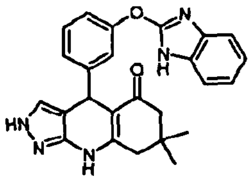 | 9 | 22 | 6692 | nd | nd | nd | nd |
| 11- comparatif | 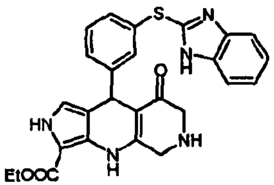 sous forme de chlorhydrate | 107 | 1755 | >10000 | nd | nd | nd | nd |

(suite)

| exemple | composé | IC50 AuroraB/ Incenp [nM] | IC50 Aurora A [nM] | IC50 HeLa [nM] | IC50 HCT116 [nM] | IC50 PDE3 [nM] | microsome humain [%] | microsome souris [%] |
|---|---|---|---|---|---|---|---|---|
| **12-comparatif** | 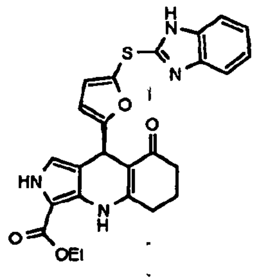 | | | | | | | |
| **13-comparatif** | 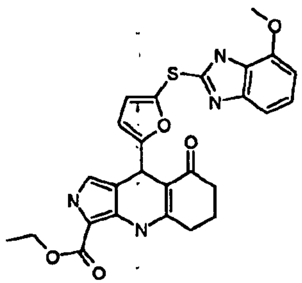 | 10 | 11 | 20 | nd | 93% inhibition à 1000 nM | 53% | 74% |
| nd : non déterminé | | | | | | | | |

**Tableau II**

| Composé | HCl 0,05N eau/acétonitrile 50/50 | NaOH 0,05N eau/acétonitrile 50/50 | Tampon phosphate 25 mM pH=7,4 eau/acétonitrile 50/50 | Tampon phosphate 25 mM pH=7,4 + 1% benzylamine eau/acétonitrile 50/50 | Tampon phosphate 25 mM pH=7,4 + 1% 2-mercaptoéthanol eau/acétonitrile 50/50 |
|---|---|---|---|---|---|
| composé 12 (comparatif) | stable | stable | stable | stable | instable (temps de demie-vie $T_{1/2}$=2,06 h) |
| composé 2 (composé lévogyre (Ia)) | stable | stable | stable | stable | stable |

**Tableau III**

| exemple | Métabolisme mesuré en présence de fraction microsomale humaine | Métabolisme mesuré en présence de fraction microsomale murine |
|---|---|---|
| composé 12 (comparatif) | 75% | 81% |
| composé 2 (composé lévogyre (Ia)) | 32% | 50% |

**Tableau IV**

| **Exemple** | **Clairance intrinsèque mesurée en présence d'hépatocytes humains (en ml.$h^{-1}.10^{-6}$ cellules)** |
|---|---|
| **composé 12 (comparatif)** | **0,29** (moyenne de 3 déterminations obtenues avec 3 préparations différentes d'hépatocytes) |
| **composé 2 (composé lévogyre (Ia))** | **0,121** (moyenne de 5 déterminations obtenues avec 5 préparations différentes d'hépatocytes) |

**Revendications**

1. Composé de formule (I) :

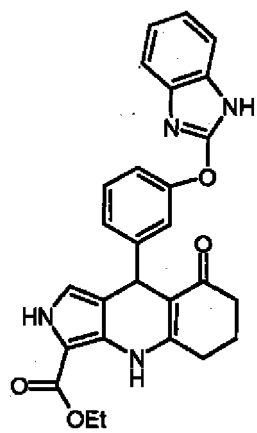

**(I)**

2. Composé selon la revendication 1 sous forme lévogyre.

**3.** Composé selon la revendication 2 présentant un pouvoir rotatoire $[\alpha]_D$= -38,6±0,7 à une concentration de 0,698 mg/ml dans le méthanol.

**4.** Composé selon l'une quelconque des revendications 1 à 3 sous forme de base ou d'un sel d'addition à un acide, notamment un acide pharmaceutiquement acceptable.

**5.** Composé selon l'une quelconque des revendications 1 à 4 en tant qu'inhibiteur sélectif des kinases Aurora A et B.

**6.** Composé selon l'une quelconque des revendications 1 à 5 en tant qu'anticancéreux.

**7.** Utilisation du composé selon l'une quelconque des revendications 1 à 5 pour la préparation d'un médicament pour le traitement d'un cancer.

**8.** Composition pharmaceutique comprenant le composé selon l'une quelconque des revendications 1 à 6 ainsi qu'au moins un excipient pharmaceutiquement acceptable.

**9.** Médicament comprenant le composé selon l'une quelconque des revendications 1 à 6.

**10.** Procédé d'obtention d'un composé selon l'une quelconque des revendications 1 à 4 consistant à :

■ faire réagir ensemble les 3 composés suivants, PG désignant un groupe protecteur de la fonction NH du benzimidazole

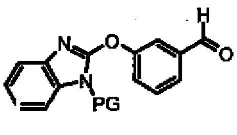 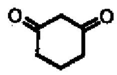 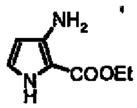

pour obtenir le composé :

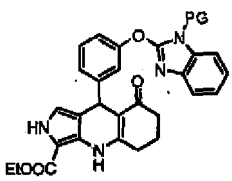 ;

■ à déprotéger la fonction NH du benzimidazole pour obtenir le composé de formule (I) ;
■ le cas échéant, à séparer le composé lévogyre.

**11.** Procédé selon la revendication 10 dans lequel la réaction entre les 3 composés est conduite dans un alcool au reflux, notamment le 1-butanol.

**12.** Procédé selon l'une des revendications 10 à 11 dans lequel PG représente le groupe

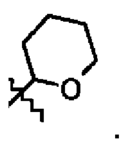 .

**13.** Composé choisi dans la liste suivante :

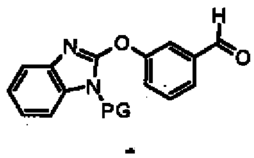
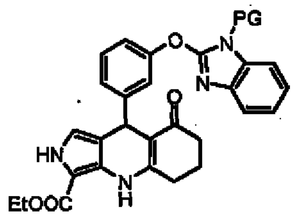

PG désignant un groupe protecteur de la fonction NH du benzimidazole.

**14.** Composé selon la revendication 13 dans lequel PG représente le groupe

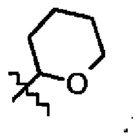

**15.** Utilisation d'un composé tel que défini à la revendication 13 ou 14 en tant qu'intermédiaire dans la préparation d'un composé selon une quelconque des revendications 1 à 4.

**Patentansprüche**

**1.** Verbindung der Formol (I):

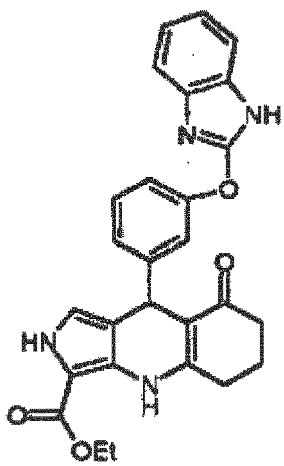

(I)

**2.** Verbindung nach Anspruch 1 in linksdrehender Form.

**3.** Verbindung nach Anspruch 2 mit einer optischen Drehung $[\alpha]_D$ = -38,6 ± 0,7 bei einer Konzentration von 0,698 mg/ml in Methanol.

**4.** Verbindung nach einem der Ansprüche 1 bis 3 in Basenform oder in Form eines Additionssalzes mit eimer Säure, insbesondere einer pharmazeutisch unbedenklichen Säure.

**5.** Verbindung nach einen der Ansprüche 1 bis 4 als selektiver Aurora-A- und Aurora-B-Kinase-Inhibitor.

**6.** Verbindung nach einem der Ansprüche 1 bis 5 als Antikrebsmittel.

**7.** Verwerdung der Verbindung nach einem der Ansprüche 1 bis 5 zur Herstellung eines Medikaments der Behandlung einer Krebserkrankung.

**8.** Pharmazeutische Zusammensetzung, umfassend die Verbindung nach einem der Ansprüche 1 bis 6 sowie mindestens einen pharmazeutisch unbedenklichen Hilfsstoff.

**9.** Medikament, umfassend die Verbindung nach einem der Ansprüche 1 bis 6.

**10.** Verwahren zum Erhalt einer Verbindung nach einem der Ansprüche 1 bis 4, bei dem man:

■ die 3 folgenden Verwindungen miteinander umsetzt, wobei PG für eine Schutzgruppe der NH-Funktion des Benzimidazols steht:

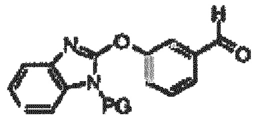
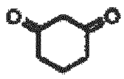
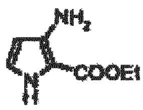

wobei man die Verwindung:

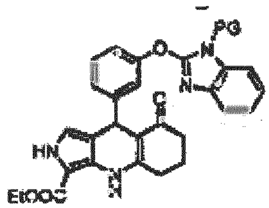

verhält;
■ die NH-Funktion des Benzimidazols entschützt, wobei man die Verbindung der Formel (I) erhält;
■ gegebenenfalls die linksdrehende Verbindung abtrennt.

**11.** Verwahren nach Anspruch 10, bei dem man die Umsetzung zwischen den 3 Verwindungen in einem Alkohol unter Rückfluss, insbesondere 1-Butanol, durchführt.

**12.** Vorfahren nach einem der Ansprüche 10 bis 11, bei dem PG für die Gruppe

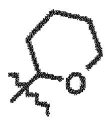

steht.

**13.** Verbindung, ausgewählt aus der folgenden Liste:

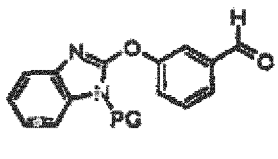
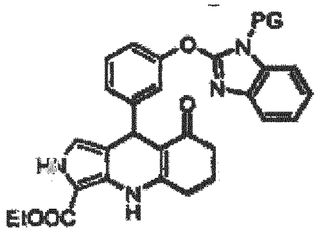

wobei PG für eine Schutzgruppe der NH-Funktion des Benzimidazols steht.

**14.** Verbindung nach Anspruch 13, wobei PG für die

Gruppe

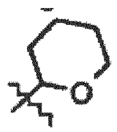

steht.

**15.** Verwendung einer Verbindung gemäß Anspruch 13 oder 14 als Zwischenprodukt bei der Herstellung einer Verbindung nach einem der Ansprüche 1 bis 4.

## Claims

**1.** Compound of formula (I):

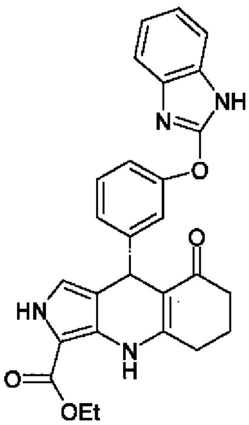

(I)

**2.** Compound according to Claim 1 in levogyratory form.

**3.** Compound according to Claim 2, exhibiting an optical rotation $[\alpha]_D$= -38.6±0.7 at a concentration of 0.698 mg/ml in methanol.

**4.** Compound according to any one of Claims 1 to 3, in the form of a base or addition salt with an acid, particularly a pharmaceutically acceptable acid.

**5.** Compound according to any one of Claims 1 to 4, as a selective inhibitor of Aurora A and B kinases.

**6.** Compound according to any one of Claims 1 to 5, as an anti-cancer.

**7.** Use of the compound according to any one of Claims 1 to 5 for preparing a medicament for treating a cancer.

**8.** Pharmaceutical composition comprising the compound according to any one of Claims 1 to 6 and at least one pharmaceutically acceptable excipient.

**9.** Medicament comprising the compound according to any one of Claims 1 to 6.

**10.** Process for preparing a compound according to any one of Claims 1 to 4, consisting in:

■ reacting together the three components below, PG denoting a protective group for the NH function of the benzimidazole

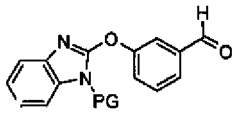 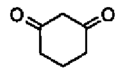 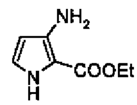

to give the compound:

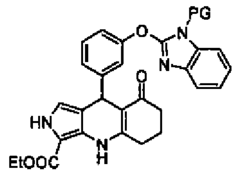;

■ deprotecting the NH function of the benzimidazole, to give the compound of formula (I);
■ where appropriate, isolating the levogyratory compound.

**11.** Process according to Claim 10, wherein the reaction between the three compounds is carried out in an alcohol at reflux, particularly 1-butanol.

**12.** Process according to one of Claims 10 to 11, wherein PG represents the group

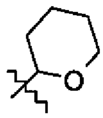.

**13.** Compound selected from the following list:

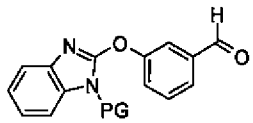 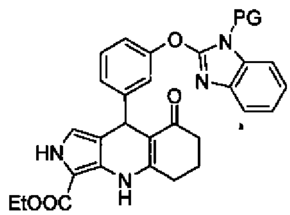

where PG denotes a protective group for the NH function of the benzimidazole.

**14.** Compound according to Claim 13, wherein PG represents the group

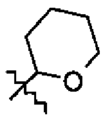.

**15.** Use of a compound as defined in Claim 13 or 14 as an intermediate in the preparation of a compound according to any one of Claims 1 to 4.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 0136422 A
- WO 2004005323 A
- WO 2005016245 A
- WO 2007012972 A
- EP 1746097 A
- WO 02062795 A


### Littérature non-brevet citée dans la description


- *Nature Reviews,* 2004, vol. 4, 927-936
- *Cancer Res.,* 2002, vol. 94, 1320
- *Oncogene,* 2002, vol. 21, 6175
- *Mol.Cell.Biol.,* 2009, vol. 29 (4), 1059-1071
- *Expert Opin. Ther. Patents,* 2005, vol. 15 (9), 1169-1182
- *Clin.Cancer Res.,* 2008, vol. 14 (6), 1639
- Inhibitors of PDE3 as adjunct therapy for dilated cardiomyopathy. *Exp.Opin.Invest.drugs,* 2002, vol. 11, 1529-1536
- What is wrong with positive inotropic drugs ? Lessons from basic science and clinical trials. *Eur. Heart J. supplements,* 2002, vol. 4, D43-D49
- Chimie pharmaceutique. 2003
- Why drugs fail-a study on side effects in new chemical entities. *Curr. Pharm.,* 2005, vol. 11, 3545
- Human hepatocytes in primary culture: the choice to investigate drug metabolism in man. *Curr.Drug Metab.,* 2004, vol. 5 (5), 443-462
- *Bioorg.Med.Chem.Lett.,* 2002, vol. 2, 1481-1484
- **P.STAHL ; C.WERMUTH.** Handbook of Pharmaceutical Salts. Wiley
- **BENDER, A.T. ; BEAVO, J.A.** *Pharmacol Rev.,* 2006, vol. 58, 488-520